# EUROPÄISCHE PATENTSCHRIFT

(11) **EP 0 321 442 B1**
(45) Veröffentlichungstag und Bekanntmachung des Hinweises auf die Patenterteilung: **05.05.1993**
(21) Anmeldenummer: 88890314.3
(22) Anmeldetag: 12.12.1988
(51) Int. Cl.: A61L 27/00, A61L 25/00

(54) **Biologisch resorbierbares Implantationsmaterial sowie Verfahren zur Herstellung desselben**
Biologically absorbable implant material, and method for its preparation
Matière d'implantation biologiquement absorbable ainsi que procédé pour sa préparation

(30) Priorität: 17.12.1987 AT 3337/87
(43) Veröffentlichungstag der Anmeldung: 21.06.1989
(73) Patentinhaber: IMMUNO Aktiengesellschaft, A-1221 Wien (AT)
(72) Erfinder: Redl, Heinz, Dr., A-1060 Wien (AT); Schlag, Günther, Dr., A-1190 Wien (AT); Pridun, Nestor, Dr., A-1130 Wien (AT)
(74) Vertreter: Wolfram, Gustav, Dipl.-Ing.

(56) Entgegenhaltungen:
- GB-A- 2 175 506
- GB-A- 2 175 507
- GB-A- 2 175 807
- US-A- 4 394 370
- JOURNAL OF SURGICAL RESEARCH, vol. 37, 1984, pages 487-496, Academic Press, Inc.; J.B. MULLIKEN et al.: "Induced osteogenesis - the biological principle and clinical applications"

## Beschreibung

Die Erfindung betrifft ein biologisches resorbierbares Implantationsmaterial zur Füllung bzw. Schließung von Weichgewebehöhlen und zum Ersatz von Weichteilgeweben sowie ein Verfahren zur Herstellung desselben.

Im Bereich der Orthopädie sind Implantationsmaterialien zur Auffüllung von Knochenhöhlen bekannt, welche durch teilweise Enteiweißung und Denaturierung des Restproteins aus Spongiosa-Knochengewebe hergestellt werden (DE-C 961 654). Dieses unter dem Namen "Kieler Spongiosa" bekannte Material hat im wesentlichen den gleichen Kalziumgehalt wie native Spongiosa. Es ist daher hart und völlig unelastisch und für eine Applikation in Weichteilgeweben ungeeignet.

In der Knochenchirurgie ist weiters ein aus Corticalis-Knochen, d.h. Röhrenknochen, durch Entkalken hergestelltes Material bekannt (Journal of Surgical Research 37, 487 - 496 (1984), welches eine osteoinduktive Wirkung aufweist, die seinem Gehalt an "Bone Morphogenetic Protein" zugeschrieben wird. Wegen dieser osteoinduktiven Eigenschaft ist ein Einsatz außerhalb eines Knochens kontraindiziert.

In der EP-A-0 171 176 ist eine Komposition zur Behandlung von Knochendefekten beschrieben, welche aus Knochenkollagen durch Entkalken und Lyophilisieren hergestellt wird und nach Rekonstitution ein Gel bildet. Wegen der erwünschten osteoinduktiven Wirkung dieses Materials wird darauf geachtet, daß keine Vernetzung des Kollagens während der Präparierung stattfindet.

Weiters ist aus der DE-B- 28 54 490 ein Knochenersatzmaterial auf Basis von Kollagen bekannt, welches aus Knochen durch Entfernen des Blutfarbstoffes und anderer wasserlöslicher Eiweiße, Entfetten und Herauslösen der Mineralanteile durch Komplexbildner oder Ionenaustauscher und abschließendem Gefriertrocknen hergestellt ist. Das Material ist nach Rekonstitution ungenügend elastisch und zur Applikation in Weichteilgeweben nicht geeignet.

Zur Applikation an oder in Weichteilgeweben sind bisher nur Kollagenvliese als Abdeckungs- und Implantationsmaterialien bekannt geworden. Diese Vliese besitzen allerdings im feuchten Zustand keine Formstabilität und keine Elastizität; sie sind für eine feste Verankerung in einem bewegten Gewebe, z. B. in der Lunge, nur bedingt geeignet. Synthetische Materialien, wie Acrylate, die ebenfalls vorgeschlagen worden sind, haben wieder andere Nachteile, von denen massiver Fremdkörperreiz, Abkapselung, mangelnde Einheilung und mögliche Freisetzung von toxischen Abbau-und Nebenprodukten im Vordergrund stehen.

Als Stand der Technik sind auch prothetische Implantationsmaterialien bekannt, die aus Körpergeweben durch Behandlung mit Proteinvernetzungsmitteln hergestellt werden. Solche Materialien werden verwendet, um die Standfestigkeit bestimmter Organe, z. B. von Herzklappen oder von Gefäßprothesen, zu erhöhen. Solche Implantate sind z. B. in der EP-B-0 174 737 und in der US-A-4 120 649 beschrieben.

Die Erfindung stellt sich die Aufgabe, ein gut verträgliches Implantationsmaterial zu schaffen, das einerseits zur Schließung von Gewebehöhlen, anderseits aber auch zum Ersatz von bestimmten Gewebeteilen verwendet werden kann und die nachteiligen bzw. den therapeutischen Einsatz begrenzenden Eigenschaften der bekannten Produkte nicht aufweist.

Nach Lungenresektionen bzw. auch nach anderen Erkrankungen der Lunge können bronchopleurale Fisteln auftreten. Diese sind septische Öffnungen oder Höhlen zwischen dem Bronchialbaum und der Pleura, die mit Sekret bzw. Eiter gefüllt sind. Sie werden beim Atmen und Husten fortlaufend von Luft und Sekreten durchströmt, ihre Größe kann wenige Millimeter bis mehrere Zentimeter im Durchmesser betragen. Es ist bisher ein ungelöstes Problem in der Thoraxchirurgie, solche Bronchusfisteln zu verschließen und zur Abheilung zu bringen, besonders wenn die Einbringung des Implantates auf endoskopischem Weg erfolgen soll, was der schnellste und schonendste Weg wäre. An ein Implantationsmaterial, welches endoskopisch eingebracht und fixiert werden soll, werden besondere Anforderungen gestellt. Es muß einerseits eine mechanische Verspreizung ermöglichen, anderseits muß es durch den Bronchialbaum an die Fistel herangebracht werden können.

Das Implantat muß verformbar und komprimierbar sein. Es muß in Anwesenheit von Feuchtigkeit seine ursprüngliche Form wieder annehmen können, d. h. einen hohen Memory-Effekt besitzen. Weiters muß das Material eine erhebliche Festigkeit aufweisen, aber doch resorbierbar bleiben, da an nicht resorbierbaren Materialien Keime haften bleiben und immer wieder zu Abszessen oder neuen Fisteln führen können. Außerdem soll es nach einem weiteren Aspekt der Erfindung möglich sein, nach Einbringen des Implantates einen sofortigen gas- und flüssigkeitsdichten Verschluß zu erzielen. Schließlich soll es möglich sein, in das Material bakterizide bzw. die Wundheilung stimulierende oder sonstige pharmazeutische Substanzen zu inkorporieren.

Die Erfindung, mit der diese Aufgaben gelöst werden, besteht bei einem biologischen, resorbierbaren Implantationsmaterial zur Füllung bzw. Schließung von Weichgewebehöhlen und zum Ersatz von Weichteilgeweben darin, daß es
- aus zu Formkörpern geformtem Knochengewebe menschlicher bzw. tierischer Herkunft besteht, welches
- entkalkt ist,
- dessen native Proteine mit einem Proteinvernetzungsmittel vernetzt sind, um eine osteoinduktive Wirkung zu vermeiden,
- wobei die Formkörper eine hohe Elastizität bei niedriger Hysterese zwischen belastetem und unbelastetem Zustand aufweisen.
Erfindungsgemäß wird also der ursprüngliche Proteingehalt der entkalkten Knochenhartsubstanz belassen und die hohe Elastizität und Beständigkeit durch die Vernetzung der nativen Proteine bewirkt.

Die aus Knochengewebe geformten Formkörper können beispielsweise Blöckchen-, Kegel- oder Kugelform aufweisen.

Zum Ersatz von röhrenförmigen Weichteilen, wie Trachea-Stücken wird das Implantationsmaterial vorzugsweise aus Corticalis-Knochenteilen hergestellt.

Zur Füllung bzw. Schließung von Gewebehöhlen kann das Implantationsmaterial aus Spongiosa-Knochenteilen hergestellt sein und schwammartige Konsistenz aufweisen, wobei das Porenvolumen des Materials 55 - 95 % betragen soll. Das Material ist in trockenem Zustand komprimierbar; in Gegenwart von Feuchtigkeit nimmt es jedoch die ursprüngliche Form wieder ein, d. h. es weist einen ausgeprägten "Memory-Effekt" auf.

Die Entkalkung wird soweit durchgeführt, daß der Restkalziumgehalt max. 80 mMol/g Feuchtgewicht beträgt.

Nach einer bevorzugten Ausführungsform kann das Implantationsmaterial einen Gewebeklebstoff auf Fibrinogen-oder Kollagenbasis enthalten.

Die Erfindung umfaßt weiters ein Verfahren zur Herstellung des Implantationsmaterials, welches dadurch gekennzeichnet ist, daß natives proteinhältiges Knochenmaterial mit einem Entkalkungsmittel entkalkt, vom Entkalkungsmittel freigewaschen, sodann mit einem Proteinvernetzungsmittel behandelt, zur Entfernung des Proteinvernetzungsmittels wiederholt gewaschen, gegebenenfalls mit pharmazeutischen Lösungen, wie Lösungen von Gewebeklebstoffen, auf Fibrinogen- oder Kollagenbasis zusammengebracht, schließlich getrocknet und sterilisiert wird.

Als Vernetzungsmittel können Aldehyde, insbesondere Glutar-Aldehyd, Polyepoxidverbindungen, Diisocyanatderivate oder Carbodiimide verwendet werden.

Das Knochenmaterial kann zweckmäßigerweise vor oder nach der Entkalkung mit einem organischen Lösungsmittel, wie Chloroform-Methanol entfettet werden.

Vor oder nach der Proteinvernetzung kann auch vorgesehen werden, das Knochenmaterial mit Mitteln zu behandeln, die einen eventuellen Gehalt an unerwünschten Antigenen vermindern, z. B. Detergentien oder Enzymen.

Das Implantationsmaterial gemäß der Erfindung bzw. das Verfahren zu seiner Herstellung kann in verschiedener Weise modifiziert werden, wodurch zusätzliche pharmazeutische Wirkungen bzw. Eigenschaften erzielt werden können. So kann durch die erwähnte Mitverwendung eines Detergens, z. B. des Oktylphenoxypolyethoxyäthanol-Triton X-100 in einprozentiger Konzentration, in den Waschlösungen nicht nur der Gehalt an unerwünschten Antigenen vermindert werden, sondern es kann die Gefahr einer späteren Verkalkung des Implantates herabgesetzt werden. Weiters ist es möglich, in den Waschlösungen einen geringen Gehalt (etwa 0,5 M) an ethylendiamintetrasaurem Natrium vorzusehen, wodurch die Entkalkung verbessert wird.

Es kann auch eine Zwischenbehandlung des Implantationsmaterials mit einer Pufferlösung, die enzymatisch wirksame Substanzen enthält, beipielsweise Ficin und/oder Neuraminidase, vorgesehen werden, wodurch ebenfalls der Antigengehalt im Fertigprodukt herabgesetzt wird.

Um sicherzugehen, daß das Fertigprodukt keine Spuren von Glutaraldehyd enthält, kann das Material nach der Vernetzungsbehandlung mit einer Glycin- oder Lysinlösung (Gehalt etwa 5 mg/ml) gespült werden.

Wenn das erfindungsgemäße Implantationsmaterial aus Spongiosa hergestellt wurde, ist es zweckmäßig, es vor der Verfüllung in die Endbehälter zu komprimieren, etwa auf das halbe Volumen.

Ein besonderes Anwendungsgebiet des erfindungsgemäßen Materials ist der Ersatz von röhrchenförmigen Gewebeteilen, wie Tracheateilen. Als Ausgangsmaterial für ein solches Implantat werden Femurknochen verwendet, die mittels mechanischer Oberflächenabtragung auf der Drehbank röhrenförmig auf ca. 2 mm Wandstärke reduziert werden, bevor sie der erfindungsgemäßen kombinierten Behandlung durch Entkalken und Protein-Vernetzen unterzogen werden.

Das erfindungsgemäße Endprodukt wird, wenn es zur Füllung von Gewebehöhlen benutzt werden soll, zweckmäßig in Blöckchenform gebracht.

Das Material kann durch Gammastrahlen sterilisiert und in Behälter, die z. B. eine 70 %ige Ethanollösung enthalten, gelagert werden. Die Blöckchen werden vor der Applikation am Patienten mit Ringer-Lösung gespült.

Es ist aber auch möglich, die Blöckchen nach einer Sterilisierung mit Gammastrahlen im trockenen Zustand zu lagern, wobei gemäß einer besonders bevorzugten Ausführungsform der Erfindung die Blöckchen mit einer Fibrinkleberlösung unter sterilen Bedingungen getränkt und anschließend im Endcontainer lyophilisiert werden.

Natürlich kann das erfindungsgemäße Produkt, abgesehen von der bereits beschriebenen Blöckchen- oder Röhrenform, auch in Scheiben- bzw. in Folienform zur Verfügung gestellt werden.

Im folgenden Beispiel wird das erfindungsgemäße Verfahren bzw. das danach hergestellte Produkt näher erläutert.

1 kg Spongiosa-Knochenmaterial vom Kalb wurde durch würfeliges Zerschneiden eines Femurkopfes gewonnen.

Diese Spongiosawürfel wurden mit heißem Wasser (50°C) gespült und anschließend bei Raumtemperatur in 0,6 N Salzsäure (10 l) unter ständiger Bewegung und mehrmaligem Wechsel, Auspressen und Waschen der Spongiosablöckchen entkalkt (24 - 72 Stunden). Anschließend wurden die Blöckchen säurefrei gespült und für eine Stunde bei Raumtemperatur mit 1 % Glutar-Aldehyd in 0,05 M Phosphatpufferlösung (1 l) bei einem pH von 7,4 zwecks Vernetzung inkubiert.

Durch mindestens fünfmaliges Waschen mit einer sterilen Waschlösung und Auspressen der Spongiosa wurde der restliche Glutar-Aldehyd aus der Spongiosa entfernt. Die Blöckchen wurden gefriergetrocknet, in die Endverpackung verfüllt und darin mit 2,5 Mrad Gammastrahlen sterilisiert.

Ein so hergestelltes Implantationsmaterial in Blöckchenform wurde bei Patienten als Fistelverschluß auf endoskopischem Wege eingesetzt: Der Patient wurde mit dem starren Bronchoskop intubiert und eine Beatmung im offenen System mit dem Injekttimer durchgeführt. Nach Inspektion der Fistel wurde ein geeignetes Spongiosastück zugeschnitten und komprimiert, damit es durch das Bronchoskop durchgeführt werden konnte. Mit einer Biopsiezange wurde das Implantat in der Fistel plaziert, wo es sich durch den Kontakt mit der Bronchuswand befeuchtet, sehr rasch ausdehnte und verspreizte. Mit einem dreilumigen Sprühkatheter wurde sofort nach Setzen des Implantates 1 bis 2 ml Fibrinkleber um das Implantat aufgetropft, wodurch es weiter aufquoll und mit der Bronchuswand verklebte. Es wurde ein sofortiger luftdichter Verschluß der Fistel erreicht.

Das erfindungsgemäße Implantationsmaterial hat keine osteoinduktive Wirkung, was durch Messung von alkalischer Phosphatase bzw. durch histologische Untersuchung festgestellt werden kann. Daher kann das Material unbedenklich zur Applikation im Weichteilbereich verwendet werden.

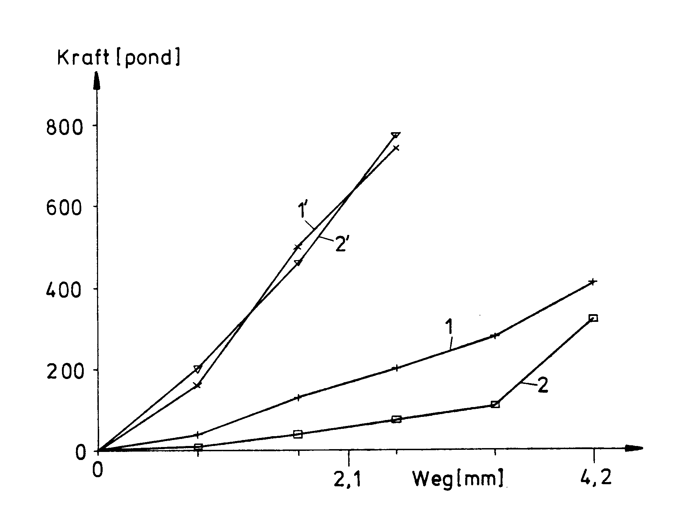

Das erfindungsgemäß hergestellte Implantationsmaterial hat hervorragende Elastizitätseigenschaften. Zum Vergleich mit bekannten Präparaten wurden Spongiosa-Blöckchen mit 1,5 cm Kantenlänge einerseits ohne Behandlung mit einem Proteinvernetzungsmittel und anderseits erfindungsgemäß durch kombinierte Entkalkung und Proteinvernetzung, wie im vorstehenden Ausführungsbeispiel beschrieben, hergestellt. Die Blöckchen wurden getrocknet und sodann mit einer 0,9 %igen NaCl-Lösung befeuchtet. Alle Blöckchen wurden in einer Belastungsmaschine (Transducer 0 - 1000 pond bei einer Belastungsgeschwindigkeit von 1 cm/min) geprüft, wobei sich Belastungs- bzw. Entlastungskurven ergaben, wie in der Zeichnung veranschaulicht.

Die Belastungskurve und die Entlastungskurve (Kraft - Weg) der Spongiosa-Blöckchen, die keiner Vernetzungsbehandlung unterzogen worden sind, sind mit 1 und 2 bezeichnet; 1′ und 2′ geben die entsprechenden Kurven der erfindungsgemäßen Präparate wieder. Es ist zu ersehen, daß bei den nicht vernetzten zum Stand der Technik gehörenden Präparaten eine verhältnismäßig niedrige Festigkeit und eine starke Hysterese auftreten, während die erfindungsgemäßen Spongiosa-Blöckchen keine Hysterese zeigen und hohe Festigkeiten aufweisen.

Die Gewebeverträglichkeit erfindungsgemäßer Spongiosa-Blöckchen mit einer Abmessung von 0,5 x 0,5 x 1 cm, hergestellt nach dem Ausführungsbeispiel, wurde in Rattenversuchen geprüft, wobei die Blöckchen subcutan am Rattenrücken implantiert, nach verschiedenen Zeiten entnommen, fixiert und histologisch aufgearbeitet wurden.

Dabei zeigte sich, daß nicht vernetzte Spongiosa-Blöckchen stärkere entzündliche Reaktionen, mehr Fremdkörperriesenzellen, mehr Fibrose und bereits nach 14 Tagen Ansätze zur Resorption zeigten und nach 4 Wochen deutliche Abbauanzeichen auftraten. Demgegenüber konnten bei erfindungsgemäß behandelten Proben keine derartigen Nebenerscheinungen festgestellt werden.

## Patentansprüche

1. Biologisches resorbierbares Implantationsmaterial menschlicher oder tierischer Herkunft zur Füllung bzw. Schließung von Weichgewebehöhlen bestehend aus zu Formkörpern geformtem Spongiosa-Knochengewebe von schwammartiger Konsistenz mit einem Porenvolumen von 55 bis 95 %, welches Material in feuchtem Zustand einen Restkalziumgehalt von maximal 80 mMol/g Feuchtgewicht aufweist; in trockenem Zustand komprimierbar ist, jedoch in Gegenwart von Feuchtigkeit die ursprüngliche Form wieder einnimmt ("Memory-Effekt"), erhältlich durch Entkalken von Spongiosa-Knochenmaterial und Behandeln mit einem Proteinvernetzungsmittel, gegebenenfalls Imprägnieren mit pharmazeutischen Lösungen, wie Lösungen von Gewebeklebstoffen auf Fibrinogen- oder Kollagenbasis, Trocknen und Sterilisieren.

2. Verfahren nach Anspruch 1, dadurch gekennzeichnet, daß das Knochenmaterial nach Behandeln mit einem Entkalkungsmittel und Vernetzen mit einem Proteinvernetzungsmittel ausgewaschen wird.

3. Verfahren nach Anspruch 1 und 2, dadurch gekennzeichnet, daß als Vernetzungsmittel Aldehyde, insbesondere Glutar-Aldehyd, Polyepoxidverbindungen, Isodicyanatderivate oder Carbodiimide verwendet werden.

4. Verfahren nach Anspruch 1 und 2, dadurch gekennzeichnet, daß das Knochenmaterial vor oder nach der Entkalkung mit einem organischen Lösungsmittel, wie Chloroform-Methanol entfettet wird.

5. Verfahren nach Anspruch 1 und 2, dadurch gekennzeichnet, daß das Knochenmaterial vor oder nach der Proteinvernetzung mit Mitteln, wie Detergentien oder Enzymen, behandelt wird, um einen Gehalt an unerwünschten Antigenen zu verringern.

## Claims

1. A biologic absorbable implant material of human or animal origin for filling and closing soft-tissue cavities, comprising spongiosa bone tissue molded to shaped bodies and having spongy consistency at a pore volume of 55 to 95 %, which material has a maximum residual calcium content of 80 mmol/g wet weight in the moist state, is compressible in the dry state, yet resumes its original shape in the presence of moisture ("memory effect"), and is obtainable by decalcifying spongiosa bone tissue and treating with a protein cross-linking agent, if desired, impregnating with pharmaceutical solutions, such as solutions of tissue adhesives based on fibrinogen or collagen, drying and sterilizing.

2. A method according to claim 1, characterized in that the bone material is washed out after treatment with a decalcifying agent and cross-linking with a protein cross-linking agent.

3. A method according to claims 1 and 2, characterized in that aldehyde, in particular glutaraldehyde, polyepoxide compounds, isodicyanate derivatives or carbodiimides are used as cross-linking agents.

4. A method according to claims 1 and 2, characterized in that the bone material is degreased with an organic solvent, such as chloroform-methanol, before or after decalcification.

5. A method according to claims 1 and 2, characterized in that the bone material, before or after protein cross-linking, is treated with agents, such as detergents or enzymes, to reduce the content of undesired antigens.

## Revendications

1. Substance à implanter, biologique, résorbable, d'origine humaine ou animale, destinée au comblement ou à la fermeture de cavités de tissus mous, composée de tissu osseux spongieux façonné en corps moulés, de consistance spongieuse, ayant un volume de pores de 55 à 95 %, laquelle substance présente une teneur résiduelle maximale en calcium de 80 mmol/g de poids à l'état humide, est comprimable à l'état sec, mais reprend sa forme originale en présence d'humidité ("memory effect"), et peut être obtenue par décalcification de substance osseuse spongieuse et traitement par un agent de réticulation des protéines, le cas échéant par imprégnation par des solutions pharmaceutiques telles que des solutions de colles tissulaires à base de fibrinogène ou de collagène, séchage et stérilisation.

2. Procédé selon la revendication 1, caractérisé en ce que la substance osseuse est rincée après traitement par un agent décalcifiant et réticulation par un agent de réticulation des protéines.

3. Procédé selon les revendications 1 et 2, caractérisé en ce qu'on utilise comme agent de réticulation des aldéhydes, notamment le glutaraldéhyde, des composés polyépoxy, des dérivés de diisocyanate ou des carbodiimides

4. Procédé selon les revendications 1 et 2, caractérisé en ce que les graisses de là substance osseuse sont éliminées avant ou après la décalcification, à l'aide d'un solvant organique tel que le chloroforme-méthanol.

5. Procédé selon les revendications 1 et 2, caractérisé en ce qu'on traite la substance osseuse, avant ou après la réticulation des protéines, à l'aide d'agents tels que des détergents ou des enzymes, pour abaisser sa teneur en antigènes indésirables.
